(19) Europäisches Patentamt
European Patent Office
Office européen des brevets

(11) Publication number : **0 516 345 A1**

(12) **EUROPEAN PATENT APPLICATION**

(21) Application number : **92304634.6**

(22) Date of filing : **21.05.92**

(51) Int. Cl.⁵ : **A61K 31/425**

(30) Priority : **28.05.91 US 706145**

(43) Date of publication of application :
**02.12.92 Bulletin 92/49**

(84) Designated Contracting States :
**AT BE CH DE DK ES FR GB GR IT LI LU NL PT SE**

(71) Applicant : **ELI LILLY AND COMPANY**
**Lilly Corporate Center**
**Indianapolis Indiana 46285 (US)**

(72) Inventor : **Cloud, Michelle Louise**
**4115 Washington Blvd.**
**Indianapolis, Indiana 46205 (US)**

(74) Representative : **Tapping, Kenneth George et al**
**Erl Wood Manor**
**Windlesham Surrey, GU20 6PH (GB)**

(54) **Use of nizatidine for the manufacture of a medicament for the treatment of reflux esophagitis.**

(57)    The present invention provides a method for treating reflux esophagitis in a mammal suffering from same which comprises administering to said mammal nizatidine in dosage amounts typically associated with conventional ulcer dosage regimens. The instantly claimed method not only provides a method for relieving the major symptoms associated with reflux esophagitis, such as heartburn, but also provides for healing of moderate-to-severe reflux esophagitis.

EP 0 516 345 A1

Gastroesophageal reflux disease (GERD) is a common problem. Some reflux is normal, and most persons with occasional heartburn, eructation, and epigastric pain obtain relief with dietary modifications, smoking cessation, antacids, or postural changes (assumption of an upright position or elevation of the head of the bed). In Western countries, the prevalence of GERD has been estimated to be from 5 to 7% of the population. Symptoms of reflux esophagitis may be severe and debilitating or somewhat minor, and there is no clear relationship between severity of reflux esophagitis and degree of symptoms.

Complications of GERD include recurrent pulmonary disease secondary to aspiration (such as bronchitis, poorly controlled asthma, and aspiration pneumonitis), chest pain that mimics angina, laryngeal disease producing chronic hoarseness, and hypopharyngeal or oral disease. In addition, complications of reflux esophagitis may include esophageal strictures, ulcer, or bleeding, and the histologic aberrations of Barrett's esophagus, the latter a premalignant condition.

The mechanisms underlying the pathophysiology of gastroesophageal reflux disease (GERD) include inadequate lower esophageal sphincter tone, abnormalities of esophageal and gastric motility, anatomical defects (such as hiatal hernia), and disordered neural mechanisms. These culminate in an imbalance between intragastric pressure and competence of the lower esophageal sphincter, coupled with impaired intraesophageal acid clearance. Acid and pepsin are the major injurious components of refluxed material.

Current therapies for treating GERD in patients who do not respond to antacids or nondrug treatments aim to decrease the acidity of the refluxate, improve esophageal and gastric motility, and/or increase lower esophageal sphincter tone. $H_2$-receptor antagonists such as cimetidine and ranitidine are highly effective in the healing of peptic ulcers. However, in contrast to their efficacy in the healing of peptic ulcers, such compounds, while generally reducing heartburn and other reflux symptoms associated with GERD, fail to significantly affect reflux esophagitis healing rates at conventional ulcer treatment dosages. In addition, long-term therapy at full dosage is required to prevent relapse if initial therapy is apparently successful.

It is an object of the present invention to provide a new method for treating reflux esophagitis, which method rapidly alleviates the major symptoms of reflux esophagitis and also heals such esophagitis, and in particular moderate-to-severe reflux esophagitis, in patients with GERD. It is a further object of the present invention to provide a method for healing reflux esophagitis and alleviating symptoms of same wherein such alleviation and healing is accomplished using dosage regimens conventionally associated with treatment of peptic ulcers. It has been found that both of the above-mentioned objects may be accomplished by using the $H_2$-receptor antagonist nizatidine, which is currently marketed under the trademark AXID for treatment of peptic ulcers. Other objects, features and advantages of the present invention will become apparent from the subsequent description and the appended claims.

The present invention provides a method of treating reflux esophagitis in a mammal, including a human, suffering from same which comprises administering nizatidine to said mammal in dosage amounts typically associated with conventional ulcer dosage regimens.

The present invention also provides the use of nizatidine for treating reflux esophagitis, and further provides the use of nizatidine in the preparation of a medicament useful for treating reflux esophagitis.

In a further embodiment, it provides an anti-reflux-esophagitis formulation comprising nizatidine, and a process for the preparation of a pharmaceutical composition which comprises admixing nizatidine prepared in a known manner with suitable inert carriers and finishing the mixture to a pharmaceutical composition suitable for the treatment of reflux esophagitis.

The method of the present invention can be used to relieve the major symptoms of reflux esophagitis, as well as heal such esophagitis, and in particular moderate-to-severe reflux esophagitis, in patients having same, at dosages lower than those previously known to be suitable for healing such condition.

As used herein, the phrase "treating reflux esophagitis" means a method of treatment which is capable of both alleviating major symptoms associated with reflux esophagitis, such as heartburn, as well as healing such esophagitis, especially moderate-to-severe reflux esophagitis. Healing of reflux esophagitis using conventional ulcer treatment dosage regimens was not previously known. As such, the ability to heal reflux esophageal ulcers is a critical aspect of the instantly claimed invention.

Furthermore, the phrase "conventional ulcer dosage regimens" means dosage amounts and frequencies of administration typically utilized by physicians in the treatment of peptic ulcers. Such amounts and frequencies of administration are well known to physicians skilled in the art of treating peptic ulcers and, as such, a further recitation thereof will not be provided. Preferred conventional ulcer dosage regimens which may be employed in the method of the present invention are those wherein nizatidine is administered twice daily. The most preferred regimen which may be employed is that wherein 150 mg of nizatidine are administered twice daily.

Nizatidine, methods for synthesizing same and pharmaceutical formulations thereof, are disclosed in United States Patent No. 4,904,792. The formulations taught in U.S. 4,904,792 can also be employed in the method of treating esophagitis provided by the instant invention. As such, the teachings of U.S. 4,904,792 with respect to methods for synthesizing nizatidine and preparing pharmaceutical formulations thereof are herein incorporated by reference.

As described above, the present invention provides a method for treating reflux esophagitis utilizing nizatidine. The method of the present invention alleviates major symptoms associated with reflux esophagitis, such as heartburn, and also heals such esophagitis, and in particular moderate-to-severe grades of same. Such alleviation and healing were demonstrated in the following studies.

Six Week Study

A total of 515 patients over the age of 18 years were enrolled in a randomized, double-blind clinical study by 58 practitioners of gastroenterology and internal medicine. Enrollment was based on the following inclusion criteria: history of GERD over the previous 3 months or longer, with symptoms (heartburn, regurgitation, retrosternal pain) present for 5 of the previous 7 days; failure of a standard antireflux regimen (including modification of diet, elevation of the head of the bed, and proper use of antacids); and endoscopic evidence of reflux esophagitis.

Women of childbearing potential who were not using an approved method of contraception were excluded, as were pregnant or lactating women. Also excluded were patients with a history of esophageal, gastric, or definitive acid-lowering surgery (except single closure of perforation); pyloric stenosis; active peptic ulceration; esophagitis or esophageal ring or stricture secondary to systemic events; concurrent serious systemic disorders (including renal or hepatic insufficiency or cancer); current treatment with ulcerogenic drug regimens; treatment with an $H_2$-receptor antagonist, prostaglandin, sucralfate, metoclopramide, bethanechol, or GAVISCON®, within 3 days before enrollment into the study; treatment with any investigational agent within the last 30 days; any condition associated with poor patient compliance (such as alcohol or drug abuse); or laboratory values on admission outside the normal range (leukocyte count <3000 GI/L; hemoglobin <6.2 mmol/L [<10 g/dL]; urea nitrogen >17.9 mmol/L [50 mg/dL]; bilirubin >25.7 mol [1.5 mg/dL], except when due to Gilbert's syndrome, documented by fractionating the bilirubin; AST [SGOT] >50 U/L or its equivalent as measured by the investigator's local laboratory).

There were 3 patient visits, 3 weeks apart, for a total of 6 weeks of treatment. Using a computer-generated randomization list, patients were assigned sequentially to receive twice daily administration of either capsules of nizatidine 150 mg, capsules of nizatidine 300 mg, or identically appearing placebo capsules. Patients received a 3-week supply of medication and GELUSIL® (200 mg aluminum hydroxide, 200 mg magnesium hydroxide, 25 mg simethicone) antacid tablets at each visit. Two GELUSIL® tablets were to be taken as needed for relief of reflux esophagitis symptoms, but not within an hour before or after taking study medication. The patients were seen and assessed for tolerability and efficacy after 3 weeks, at which time additional supplies of study medication and antacid were provided. Medication was taken for a total study duration of 6 weeks, unless endoscopy revealed that the patient's reflux esophagitis had healed, in which case the patient completed the study at visit 2 (3 weeks), or the patient was discontinued from the study for some other reason. Patients, observers, and analysts were unaware of the treatment assignment until analysis of the results was completed, except in the event of significant adverse events, death, or to protect the patients or others.

Acetaminophen was allowed for pain relief. Other antacids, other $H_2$-receptor antagonists, sucralfate, prostaglandins, anticholinergic medications, metoclopramide, and bethanechol were not allowed. If additional ulcer medications were required the patient was discontinued from the study for lack of efficacy.

Healing of reflux esophagitis was determined by endoscopic examination. GERD was confirmed by endoscopy at enrollment, and endoscopy was repeated after 3 and 6 weeks of treatment. Reflux esophagitis was graded as follows:

0 = none
1 = localized erythema of gastroesophageal junction
2 = diffuse erythema and mucosal friability
3 = erosions, diffuse erythema, and mucosal friability (moderate reflux esophagitis)
4 = frank ulceration, diffuse erythema, and mucosal friability (severe reflux esophagitis)
5 = stricture (patient was discontinued if a stricture developed during the study).

Patients with resolution of endoscopic evidence of reflux esophagitis (graded as 0) were classified as "healed."

Healing was analyzed by using an "intent-to-treat" analysis. Such method of analysis treats patients who fail to return for follow-up visits as treatment failures and thus tends to bias the healing rates downward in each group. Patients who were categorized as "healed" (not having reflux esophagitis present at endoscopic evaluation) at week 3, and then discharged from the study, were considered healed at week 6. Cumulative healing rates at 3 and 6 weeks of therapy were analyzed using a weighted, least-squares method to compare the treatment groups. In addition, changes from baseline to endpoint for ulcer-related endoscopic abnormalities (that is, gastritis, erosions, scarring, hemorrhage, Barrett's epithelium) were analyzed using Pearson's chi-square test.

The results of such study are set forth in Table 1, below. In Table 1, Column 1 discloses the week at which endoscopic evaluation was performed. Column 2 discloses the method of treatment employed (nizatidine 300 mg, nizatidine 150 mg or placebo). Columns 3 through 6 disclose the cumulative percentage of patients healed for each category of patients having a reflux esophagitis rating of from 1 to 4, respectively, at baseline (week 0). Finally, Column 7 discloses the total cumulative percentage of patients healed during the study.

## Table 1

### Cumulative percentage of patients healed (total number treated) at week 3 and week 6

| | | Reflux esophagitis at baseline | | | | |
|---|---|---|---|---|---|---|
| Week | Treatment | 1 | 2 | 3 | 4 | Total |
| 3 | Nizatidine 300 mg | 19.6% (N=51) | 28.0% (N=25) | 14.9% (N=74) | 5.6% (N=18) | 17.2% (N=169) |
| 3 | Nizatidine 150 mg | 30.2% (N=53) | 11.5% (N=26) | 18.6% (N=70) | 5.6% (N=18) | 19.6% (N=168) |
| 3 | Placebo | 19.6% (N=51) | 14.3% (N=28) | 7.5% (N=80) | 5.6% (N=18) | 11.8% (N=178) |
| 6 | Nizatidine 300 mg | 45.1% (N=51) | 56.0% (N=25) | 27.0% (N=74) | 44.4% (N=18) | 38.5% (N=169) |
| 6 | Nizatidine 150 mg | 52.8% (N=53) | 50.0% (N=26) | 38.6% (N=70) | 5.6% (N=18) | 41.1% (N=168) |
| 6 | Placebo | 41.2% (N=51) | 32.1% (N=28) | 16.3% (N=80) | 16.7% (N=18) | 25.8% (N=178) |

Alleviation of the frequency and severity of day and night symptoms of heartburn was also evaluated. Day and night symptoms of heartburn were scored by patients daily and by investigators at each 3-week visit. Five-point scales were used, with 0 representing no symptoms and 4 representing terrible, disabling symptoms (interfering with daily routine or sleep) or continual symptoms (occurring on more than 10 days since the preceding visit). Well-being was scored by patients at admission and at termination or completion of the protocol using a five-point scale, with 0 representing very poor, and 4 representing very good.

Alleviation of heartburn efficacy was assessed with analysis of variance. Three analyses were conducted: data from each visit; averages from all visits during the active phase (weeks 3 and 6); and endpoint analysis (last visit with data collection). Comparisons of efficacy involving placebo were one-sided tests. Comparisons between the two doses of nizatidine were two-sided.

The results of such test are set forth in Table 2, below. In Table 2, Column 1 discloses the symptom being evaluated (daytime or nightime heartburn). Column 2 discloses the week at which evaluation of the symptom occurred. Finally, Columns 3 and 4 disclose the treatment employed (nizatidine 300 mg, nizatidine 150 mg or placebo) and the mean severity of heartburn score obtained.

## Table 2

### Severity of Heartburn

| Symptom | Week | Treatment | Mean Severity Score |
|---|---|---|---|
| Daytime Heartburn | 0 | nizatidine 300 mg | 2.23 |
| Daytime Heartburn | 0 | nizatidine 150 mg | 2.29 |
| Daytime Heartburn | 0 | placebo | 2.30 |
| Daytime Heartburn | 3 | nizatidine 300 mg | 1.45 |
| Daytime Heartburn | 3 | nizatidine 150 mg | 1.42 |
| Daytime Heartburn | 3 | placebo | 1.76 |
| Daytime Heartburn | 6 | nizatidine 300 mg | 1.35 |
| Daytime Heartburn | 6 | nizatidine 150 mg | 1.36 |
| Daytime Heartburn | 6 | placebo | 1.50 |
| Nightime Heartburn | 0 | nizatidine 300 mg | 2.12 |
| Nightime Heartburn | 0 | nizatidine 150 mg | 2.02 |
| Nightime Heartburn | 0 | placebo | 2.17 |
| Nightime Heartburn | 3 | nizatidine 300 mg | 1.33 |
| Nightime Heartburn | 3 | nizatidine 150 mg | 1.34 |
| Nightime Heartburn | 3 | placebo | 1.66 |
| Nightime Heartburn | 6 | nizatidine 300 mg | 1.25 |
| Nightime Heartburn | 6 | nizatidine 150 mg | 1.21 |
| Nightime Heartburn | 6 | placebo | 1.26 |

As can be seen from the data set forth above, total cumulative healing (defined as reflux esophagitis grade 0) at 3 weeks was greater with nizatidine 150 mg than with placebo, and at 6 weeks was greater with both doses of nizatidine than placebo. Analysis was also conducted with regard to the baseline severity of reflux esophagitis. There were no differences between treatment groups for patients with grade 1 reflux esophagitis. Nizatidine 300 mg healed a greater percentage of patients with grade 2 reflux esophagitis than did placebo at week 6. The incidence of healing was greater with nizatidine 150 mg than with placebo in grade 3 reflux esophagitis at both week 3 and week 6, and the differences between nizatidine 300 mg and placebo were nearly significant. Finally, in patients with grade 4 reflux esophagitis, the incidence of healing was greater with nizatidine 300 mg than with either nizatidine 150 mg or placebo at week 6.

Endpoint analyses of symptom severity and frequency revealed a significantly greater reduction of daytime heartburn severity with nizatidine 300 mg or 150 mg than with placebo. Heartburn frequency was also decreased with nizatidine 300 mg and nizatidine 150 mg relative to placebo. Analysis of symptoms by visit indicated superior relief of daytime heartburn with either dosage of nizatidine and superior relief of nightime heartburn with nizatidine 300 mg at the week 3 visit, compared with placebo. At week 6 there were no significant treatment differences.

Patient symptom logs were also analyzed for the first 7 days of treatment. Daytime heartburn was significantly relieved with nizatidine 150 mg after the first day and with nizatidine 300 mg after the second day compared with placebo. Nightime heartburn was significantly relieved with nizatidine 300 mg after the first day. However, in relief of nightime heartburn, nizatidine 150 mg attained statistical significance compared with placebo only on the first day of the initial treatment week.

Twelve Week Study

A total of 466 patients over the age of 18 years were enrolled in a randomized, double-blinded, clinical study by 58 practitioners of gastroenterology and internal medicine. Patient enrollment was governed by the same criteria as that set forth with respect to the six week study discussed above.

Including the initial visit, there were 5 patient visits, 3 weeks apart, for a total of 12 weeks of treatment. Using a computer-generated randomization list, patients were assigned sequentially to receive capsules of nizatidine 150 mg twice daily or nizatidine 300 mg once daily at bedtime and an identically appearing placebo capsule in the morning, or capsules of placebo twice daily. Patients received a three-week supply of medication and GELUSIL® antacid tablets at each visit. Two GELUSIL® tablets were to be taken as needed for relief of reflux esophagitis symptoms, but not within an hour before or after taking study medication. At three-weekly intervals the patients were seen and assessed for tolerability and efficacy, at which time additional supplies of study medication and antacid tablets were provided. Medication was taken for the total study duration of 12 weeks unless endoscopy revealed that the patient's reflux esophagitis had healed (in which case the patient completed the study at visit 3 or 6 weeks or the patient discontinued the study for some other reason. Patients, observers, and analysts were unaware of the treatment assignment until analysis of the results was completed.

Acetaminophen was allowed for pain relief. Other antacids, other $H_2$-receptor antagonists, sucralfate, prostaglandins, anticholinergic medications, metoclopramide, and bethanechol were not allowed. If additional ulcer medications were required for exacerbation of ulcer symptoms, the patient was discontinued from the study for lack of efficacy.

Healing of reflux esophagitis was again determined by endoscopic examination. GERD was confirmed by endoscopy at enrollment, and endoscopy was repeated after 6 and 12 weeks of treatment. Reflux esophagitis was graded using the same scale described previously. Patients with resolution of endoscopic evidence of reflux esophagitis (graded as 0) at weeks 6 and 12 were classified as healed.

Healing was analyzed by using the "intent-to-treat" analysis discussed above. Cumulative healing rates at weeks 6 and 12 of therapy were analyzed using a weighted, least-squares method to compare the treatment groups. In addition, changes from baseline to endpoint in acid-related endoscopic abnormalities (such as gastritis, erosions, scarring, hemorrhage) were analyzed by a Pearson chi-square test.

The results of such study are set forth in Table 3, below. In Table 3, Column 1 discloses the week at which endoscopic evaluation was performed. Column 2 of Table 1 discloses the treatment employed (nizatidine 150 mg twice daily, nizatidine 300 mg once daily at bedtime or placebo). Columns 3 through 6 disclose the cumulative percentage of patients healed for each category of patients having a reflux esophagitis rating of from 1 to 4, respectively, at baseline (week 0). Finally, Column 7 discloses the total cumulative percentage of patients healed during the study.

## Table 3

## Cumulative percentage of patients healed (total number treated) at week 6 and week 12

### Reflux esophagitis at baseline

| Week | Treatment | 1 | 2 | 3 | 4 | Total |
|---|---|---|---|---|---|---|
| 6 | Nizatidine 300 mg at bedtime | 31% (N=32) | 30% (N=23) | 10% (N=69) | 8% (N=26) | 17% (N=151) |
| 6 | Nizatidine 150 mg twice daily | 41% (N=29) | 19% (N=26) | 24% (N=68) | 16% (N=31) | 25% (N=155) |
| 6 | Placebo | 50% (N=40 | 29% (N=24) | 12% (N=65) | 7% (N=29) | 23% (N=160) |
| 12 | Nizatidine 300 mg at bedtime | 53% (N=32) | 30% (N=23) | 25% (N=69) | 12% (N=26) | 29% (N=151) |
| 12 | Nizatidine 150 mg twice daily | 55% (N=29) | 42% (N=26) | 28% (N=68) | 32% (N=31) | 36% (N=155) |
| 12 | Placebo | 60% (N=40) | 46% (N=24) | 14% (N=65) | 10% (N=29) | 29% (N=160) |

Alleviation of the frequency and severity of day and night symptoms of heartburn were also evaluated. The methods used to perform such evaluation were the same as those described in the six week study detailed above.

The results of such test are set forth in Table 4, below. In Table 4, Column 1 discloses the symptom being evaluated (daytime or nightime heartburn). Column 2 discloses the week at which evaluation of the symptom occurred. Finally, Columns 3 and 4 disclose the treatment employed (nizatidine 150 mg twice daily, nizatidine 300 mg once daily at bedtime or placebo) and the mean severity heartburn score obtained.

## Table 4

### Severity of Heartburn

| Symptom | Week | Treatment | Mean Severity Score |
|---|---|---|---|
| Daytime Heartburn | 0 | nizatidine 150 mg | 2.38 |
| Daytime Heartburn | 0 | nizatidine 300 mg | 2.23 |
| Daytime Heartburn | 0 | placebo | 2.34 |
| Daytime Heartburn | 3 | nizatidine 150 mg | 1.25 |
| Daytime Heartburn | 3 | nizatidine 300 mg | 1.58 |
| Daytime Heartburn | 3 | placebo | 1.60 |
| Daytime Heartburn | 6 | nizatidine 150 mg | 1.16 |
| Daytime Heartburn | 6 | nizatidine 300 mg | 1.31 |
| Daytime Heartburn | 6 | placebo | 1.43 |
| Daytime Heartburn | 9 | nizatidine 150 mg | 1.07 |
| Daytime Heartburn | 9 | nizatidine 300 mg | 1.29 |
| Daytime Heartburn | 9 | placebo | 1.34 |
| Daytime Heartburn | 12 | nizatidine 150 mg | 1.02 |
| Daytime Heartburn | 12 | nizatidine 300 mg | 1.10 |
| Daytime Heartburn | 12 | placebo | 1.16 |
| Nightime Heartburn | 0 | nizatidine 150 mg | 2.22 |
| Nightime Heartburn | 0 | nizatidine 300 mg | 1.96 |
| Nightime Heartburn | 0 | placebo | 2.20 |
| Nightime Heartburn | 3 | nizatidine 150 mg | 1.25 |
| Nightime Heartburn | 3 | nizatidine 300 mg | 1.58 |
| Nightime Heartburn | 3 | placebo | 1.60 |
| Nightime Heartburn | 6 | nizatidine 150 mg | 0.96 |
| Nightime Heartburn | 6 | nizatidine 300 mg | 0.87 |
| Nightime Heartburn | 6 | placebo | 1.17 |
| Nightime Heartburn | 9 | nizatidine 150 mg | 1.01 |
| Nightime Heartburn | 9 | nizatidine 300 mg | 0.87 |
| Nightime Heartburn | 9 | placebo | 1.17 |
| Nightime Heartburn | 12 | nizatidine 150 mg | 1.01 |
| Nightime Heartburn | 12 | nizatidine 300 mg | 0.87 |
| Nightime Heartburn | 12 | placebo | 1.12 |

As can be seen from the above, total cumulative healing (defined as reflux esophagitis grade 0) at weeks 6 and 12 did not differ significantly between either nizatidine treatment group or placebo. However, a nonsigni-

8

ficant trend in favor of the nizatidine 150 mg twice daily regimen was suggested. Analysis with regard to the baseline severity of reflux esophagitis revealed that at the week 12 assessment nizatidine 150 mg twice daily was significantly superior to placebo in the subgroups with moderate (grade 3) or severe (grade 4) reflux esophagitis, and at week 6 was superior to both placebo and nizatidine 300 mg once daily at bedtime in patients with moderate reflux esophagitis. Nizatidine 300 mg once daily at bedtime was not different from placebo in any of the four subgroups.

Combining baseline reflux esophagitis grades 1 and 2, and grades 3 and 4, gave larger sample sizes with which to analyze the effect of baseline mucosal damage on healing rates. After 6 weeks, nizatidine 150 mg twice daily healed a significantly greater percentage of patients with more severe mucosal damage (grades 3 and 4) than did placebo or nizatidine 300 mg once daily at bedtime. The 150 mg twice daily regimen was also superior to placebo (but not to the 300 mg once daily regimen) at week 12.

Endpoint analyses of symptom severity and frequency revealed a significantly greater reduction of daytime heartburn severity with nizatidine 150 mg twice daily than with nizatidine 300 mg once daily at bedtime or placebo. The frequency of heartburn pain with nizatidine 150 mg twice daily versus placebo almost attained statistical significance. Analysis of heartburn by visit indicated superior relief of daytime heartburn with nizatidine 150 mg twice daily at most visits, and of nightime heartburn relative to placebo at week 3.

Patients' daily logs were analyzed for the first 7 days for daytime and nightime heartburn. Relative to placebo, significant relief of daytime heartburn was achieved with nizatidine 150 mg twice daily beginning after the first day of treatment, and of nightime heartburn beginning after the second day of treatment. Nizatidine 150 mg twice daily was also superior to nizatidine 300 mg once daily at bedtime in consistently relieving daytime heartburn and in relieving nightime heartburn after 5 days of therapy.

## Claims

1. The use of nizatidine in the preparation of a medicament for treating reflux esophagitis using conventional ulcer dosage regimens.

**European Patent Office**

## EUROPEAN SEARCH REPORT

Application Number

EP   92 30 4634

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | AM.J.GASTROENTEROL. vol. 83, no. 9, 1988, page 1019; M.CLOUD ET AL.: 'Nizatidine 150 mg b.i.d relieves symptoms and decreases severity of esophagitis in gastro-esophageal reflux' *whole abstract* | 1 | A61K31/425 |
| X | DRUGS vol. 36, no. 5, 1988, pages 521 - 539; A.H.PRICE ET AL.: 'Nizatidine' * page 535, paragraph 3.4 * | 1 | |

**TECHNICAL FIELDS SEARCHED (Int. Cl.5 )**

A61K

The present search report has been drawn up for all claims

| Place of search | Date of completion of the search | Examiner |
|---|---|---|
| MUNICH | 12 AUGUST 1992 | TZSCHOPPE D.A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document